# EUROPEAN PATENT APPLICATION

(11) **EP 4 781 992 A1**
(43) Date of publication of application: **29.07.2026**
(21) Application number: 24867596.9
(22) Date of filing: 23.09.2024
(51) Int. Cl.: A61K 31/522, C07D 473/32, A61P 35/00

(54) **USE OF IMIDAZOLIDINONE DERIVATIVE IN PREPARATION OF DRUG FOR TREATING TUMORS IN COMBINATION WITH RADIOTHERAPY, AND TREATMENT METHOD**

(30) Priority: 21.09.2023 CN 202311227143; 28.03.2024 CN 202410365297; 15.05.2024 CN 202410603540
(71) Applicant: Kangbaida (Sichuan) Biotechnology Co., Ltd., Chengdu, Sichuan 611137 (CN)
(72) Inventor: LIU, Rui, Chengdu, Sichuan 610000 (CN); WEI, Yonggang, Chengdu, Sichuan 610000 (CN); SUN, Yi, Chengdu, Sichuan 610000 (CN)
(74) Representative: Carpmaels & Ransford LLP
(86) International application number: PCT/CN2024/120290
(87) International publication number: WO 2025/061191

(57) **Abstract**

The present invention provides the use of a compound represented by general formula (I) or a stereoisomer, solvate, prodrug, metabolite, pharmaceutically acceptable salt or co-crystal thereof as an active ingredient in the preparation of a drug for use in the treatment of a tumor in combination with radiotherapy

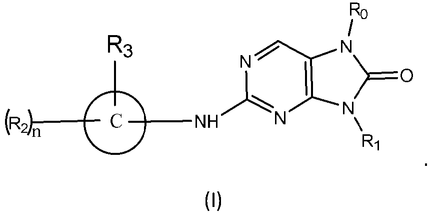

## Description

### TECHNICAL FIELD

The present invention relates to the use of an imidazolinone derivative in the preparation of a drug for use in the treatment a tumor in combination with radiotherapy.

### BACKGROUND ART

Radiotherapy is one of the most important methods for the comprehensive treatment of malignant tumors, and the neoadjuvant therapy based on conventional long-term radiotherapy has become a standard regimen for treating various malignant tumors. However, long-term clinical studies have found that the responses of patients with malignant tumors to radiotherapy vary greatly. Although some patients can achieve complete pathological remission at the end of a treatment cycle, there are still some patients who can not benefit from long-term treatment, and a few even develop worsening progress. Studies have found that the enhanced DNA damage repair mechanism due to radiotherapy is an important reason for the resistance mechanism of tumors to radiotherapy.

DNA double-strand break (DSB) is a highly harmful form of DNA damage in cells, and DNA double-strand breaks that are not repaired in time are closely associated with the canceration of cells. Non-homologous end-joining (NHEJ) is one of the main pathways for the repair of DNA double-strand breaks in cells. In NHEJ, the DSB end is first recognized and bound by Ku70/80, which then binds to a DNA-dependent protein kinase catalytic subunit (DNA-PKcs) to form a DNA-dependent protein kinase (DNAPK), i.e., an NHEJ initiation complex (DNAPK). Subsequently, two DNAPKs bind to the ends of damaged DNA while recruiting subsequent NHEJ repair factors (XRCC4 and XLF) and DNA ligase IV (LiglV) to repair the damaged DNA.

WO 2021209055 discloses the use of an imidazolinone derivative in the preparation of a drug for use in the treatment of a cancer, in which the compounds described in the specification have high selectivity and significant inhibitory activity against DNA-PK.

### SUMMARY OF THE INVENTION

Herein, the inventors of the present invention aim to increase the sensitivity of tumors to radiotherapy by combining the prior art with radiotherapy to overcome the deficiencies of the prior art.

An objective of the present invention is to provide the use of an imidazolinone derivative for use in the treatment of a tumor in combination with radiotherapy, so as to overcome the deficiencies of the prior art.

In one or more embodiments of the present application, there is provided the use of a compound of general formula (I), or a stereoisomer, solvate, prodrug, metabolite, pharmaceutically acceptable salt or co-crystal thereof as an active ingredient in the preparation of a drug for use in the treatment of a tumor in combination with radiotherapy, wherein the tumor is selected from esophageal cancer, lung cancer, head and neck cancer, gastric cancer, pleural mesothelioma, thymic carcinoma, kidney cancer, bladder cancer, hepatocellular carcinoma, colorectal cancer, lymphoma, ovarian cancer, breast cancer, fibrosarcoma, myosarcoma, liposarcoma, chondrosarcoma, osteoblastic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, pancreatic cancer, prostate cancer, squamous cell carcinoma, basal cell carcinoma, adenocarcinoma, sweat gland carcinoma, sebaceous carcinoma, papillary carcinoma, papillary adenocarcinoma, cystadenocarcinoma, medullary carcinoma, bronchogenic carcinoma, hepatocellular carcinoma, cholangiocarcinoma, choriocarcinoma, seminoma, embryonal carcinoma, Wilms' tumor, cervical cancer, testicular tumor, small cell lung cancer, epithelial cancer, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma, leukemia, chronic leukemia, polycythemia vera, multiple myeloma, Waldenstrom's macroglobulinemia or heavy chain disease, or any combination thereof: 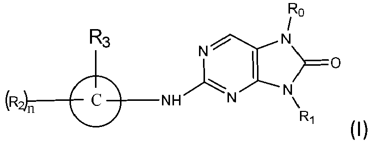 wherein 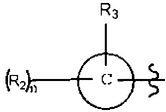 is 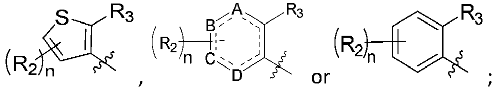
-̅ -̅ -̅ is a single bond or double bond;
in 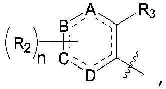 A, B, C, D are each independently C or N, and at least one of A, B, C and D is N;
R₀ is H, C₁₋₆ alkyl or cyclopropyl, wherein the C₁₋₆ alkyl is optionally further substituted with one or more substituents selected from halogen and deuterium;
R₁ is 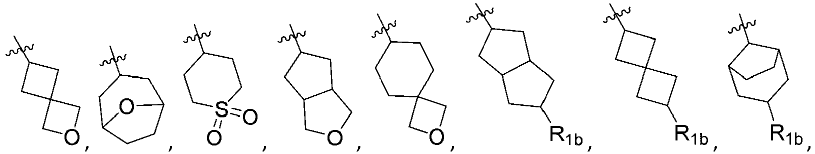 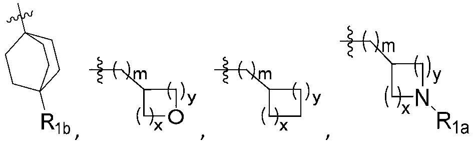 or pyridyl, and R₁ is optionally further substituted with 1 or 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
R₂ is H, cyano, =O, carboxyl, -C(=O)NR₂ₐR_{2b}, C₁₋₆ alkoxy, C₁₋₆ alkyl, halogen, -S(=O)₂R₂ₐ or - C(=O)OC₁₋₆ alkyl, wherein the C₁₋₆ alkyl, -C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atoms to which they are attached form a 5- to 6-membered heterocyclyl, which contains 1, 2 or 3 heteroatoms selected from N, O and S and is optionally further substituted with one or more substituents selected from C₁₋₆ alkyl, OH and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1 or 2; and
x and y are each independently 1, 2 or 3;
with the provisos that
when 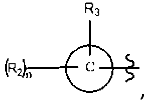 R₀, R₂, and R₃ simultaneously satisfy the following conditions, R₁ is not 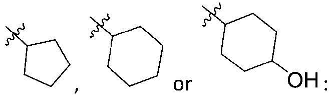 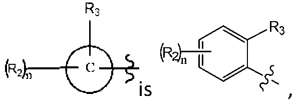 n is 1, R₀ is H or methyl, R₂ is methoxy or -S(=O)₂Me, and R₃ is methyl.

In one or more embodiments of the present application, R₁ is 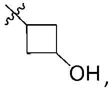 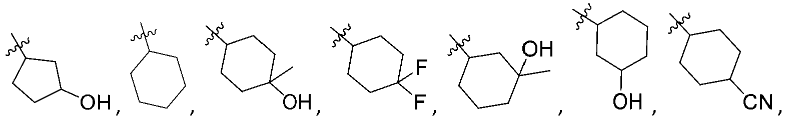 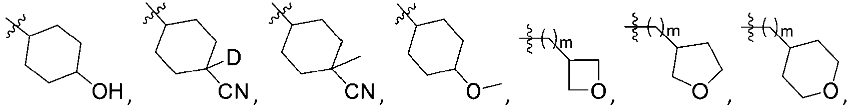 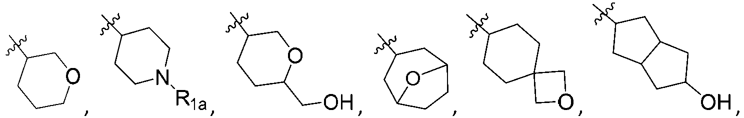 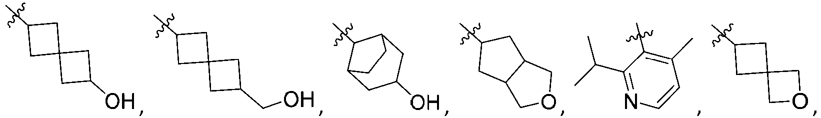 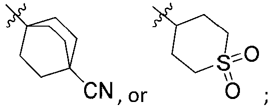
R₁ₐ is H or C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl, wherein the - C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D or halogen;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from OH and halogen;
R₃ is halogen or C₁₋₆ alkyl, wherein the C₁₋₆ alkyl is optionally further substituted with 1 to 3 substituents selected from D and halogen;
m is 0 or 1; and
n is 0, 1 or 2;
with the provisos that
when 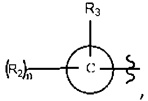 R₀, R₂, and R₃ simultaneously satisfy the following conditions, R₁ is not 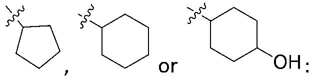 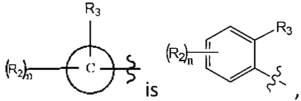 n is 1, R₀ is H or methyl, R₂ is methoxy or -S(=O)₂Me, and R₃ is methyl.

In one or more embodiments of the present application, 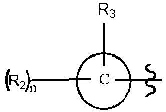 is 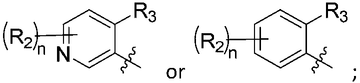
R₀ is H, C₁₋₄ alkyl or cyclopropyl, wherein the C₁₋₄ alkyl is optionally further substituted with one or more substituents selected from halogen and D;
R₁ is 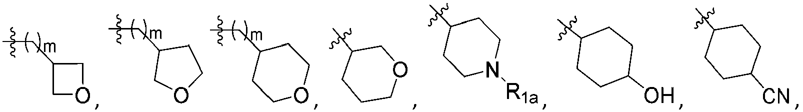 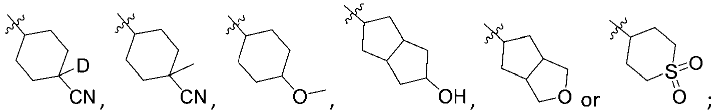
R₁ₐ is H, C₁₋₆ alkyl or -C(=O)C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl, wherein the - C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D and halogen;
alternatively, R₂ₐ and R_{2b} together with the atoms to which they are attached form a 5- to 6-membered heterocyclyl, which contains 1 to 3 heteroatoms selected from N, O and S and is also optionally further substituted with one or more substituents selected from OH and halogen;
R₃ is halogen or C₁₋₆ alkyl, wherein the C₁₋₆ alkyl is optionally further substituted with 1 to 3 substituents selected from D and halogen;
m is 0 or 1; and
n is 0, 1 or 2;
with the provisos that
when 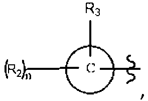 R₀, R₂, and R₃ simultaneously satisfy the following conditions, R₁ is not 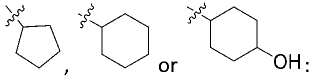
when 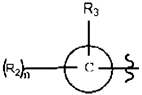 is 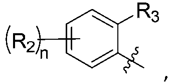 n is 1, R₀ is H or methyl, R₂ is methoxy or -S(=O)₂Me, and R₃ is methyl.

In one or more embodiments of the present application, there is provided the use of a compound of general formula (II), or a stereoisomer, solvate, prodrug, metabolite, pharmaceutically acceptable salt or co-crystal thereof as an active ingredient in the preparation of a drug for use in the treatment of a tumor in combination with radiotherapy: 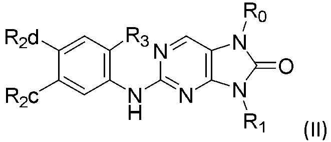 wherein
R₀ is H, C₁₋₆ alkyl or cyclopropyl, wherein the C₁₋₆ alkyl is optionally further substituted with one or more substituents selected from halogen and deuterium;
R₁ is 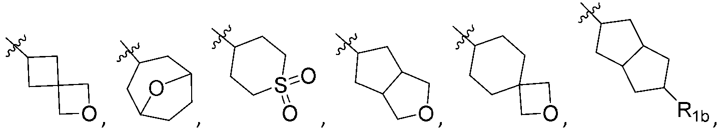 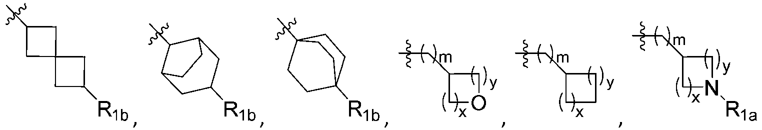 or pyridyl, and R₁ is optionally further substituted with 1 to 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
R_{2c} is H, cyano, halogen or C₁₋₆ alkoxy;
R_{2d} is H, cyano, carboxyl, -C(=O)NR₂ₐR_{2b}, C₁₋₆ alkyl, halogen, -S(=O)₂R₂ₐ or -C(=O)OC₁₋₆ alkyl, wherein the C₁₋₆ alkyl and -C(=O)OC₁₋₆ alkyl is optionally substituted with one or more substituents selected from halogen and deuterium;
R₂ₐ and R_{2b} are H, C₁₋₆ alkyl, or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from C₁₋₆ alkyl, OH and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1 or 2; and
x and y are each independently 1, 2 or 3;
with the provisos that
when 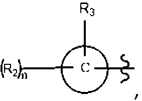 R₀, R₂, and R₃ simultaneously satisfy the following conditions, R₁ is not 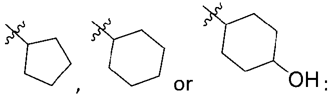 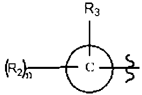 is selected from 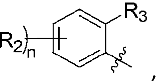 n is 1, R₀ is H or methyl, R₂ is methoxy or - S(=O)₂Me, and R₃ is methyl.

In one or more embodiments of the present application, there is provided the use of a compound of general formula (III), or a stereoisomer, solvate, prodrug, metabolite, pharmaceutically acceptable salt or co-crystal thereof as an active ingredient in the preparation of a drug for use in the treatment of a tumor in combination with radiotherapy: 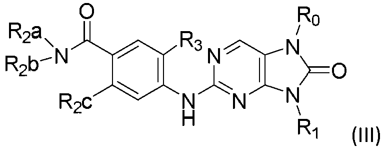 wherein
R₀ is H, C₁₋₆ alkyl or cyclopropyl, wherein the C₁₋₆ alkyl is optionally further substituted with one or more substituents selected from halogen and deuterium;
R₁ is 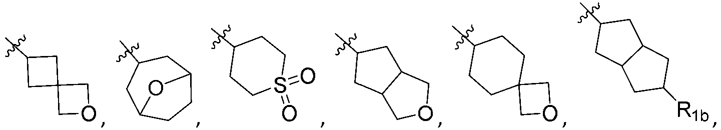 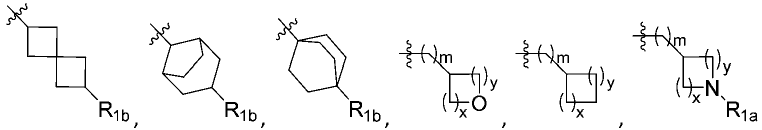 or pyridyl, and R₁ is optionally further substituted with 1 to 2 substituents selected from D, halogen, cyano, hydroxyl, C₁₋₆ alkyl and C₁₋₆ alkoxy;
R_{1b} is H, OH, cyano, or hydroxyl substituted C₁₋₆ alkyl;
R₂ₐ and R_{2b} are each independently H, C₁₋₆ alkyl, or 3- to 5-membered cycloalkyl, wherein the C₁₋₆ alkyl is optionally further substituted with one or more substituents selected from D or halogen;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from C₁₋₆ alkyl, OH and halogen;
R_{2c} is H, cyano, halogen or C₁₋₆ alkoxy, wherein the C₁₋₆ alkoxy is optionally substituted with one or more deuterium;
R₃ is halogen or C₁₋₆ alkyl, wherein the C₁₋₆ alkyl is optionally further substituted with 1 to 3 substituents selected from D or halogen;
m is 0 or 1;
n is 0, 1 or 2; and
x and y are each independently 1, 2 or 3.

In one or more embodiments of the present application, there is provided the use of a compound of general formula (IV), or a stereoisomer, solvate, prodrug, metabolite, pharmaceutically acceptable salt or co-crystal thereof as an active ingredient in the preparation of a drug for use in the treatment of a tumor in combination with radiotherapy: 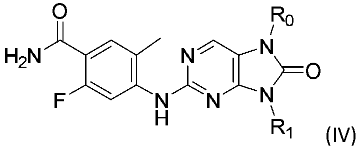 wherein
R₀ is H, C₁₋₆ alkyl or cyclopropyl, wherein the C₁₋₆ alkyl is optionally further substituted with one or more substituents selected from halogen and deuterium; and
R₁ is -(CH)ₘ-4- to 7-membered carbocyclyl, -(CH)ₘ-4- to 7-membered heterocyclyl, -(CH)ₘ-8- to 12-membered bridged ring, -(CH)ₘ-7- to 12-membered spiro ring, wherein the -(CH)ₘ-4- to 7-membered carbocyclyl, -(CH)ₘ-4- to 7-membered heterocyclyl, -(CH)ₘ-8- to 12-membered bridged ring, or -(CH)ₘ-7- to 12-membered spiro ring is optionally further substituted with one or more substituents selected from hydroxy, cyano, halogen, =O, C₁₋₆ alkyl, C₁₋₆ alkoxy, and hydroxy substituted C₁₋₆ alkyl.

In one or more embodiments of the present application,
R₀ is H, C₁₋₆ alkyl or cyclopropyl, wherein the C₁₋₆ alkyl is optionally further substituted with one or more substituents selected from halogen and deuterium;
R₁ is 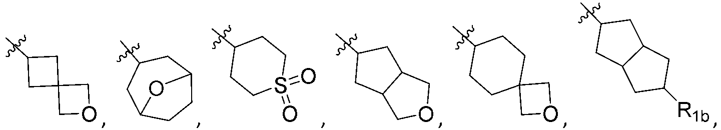 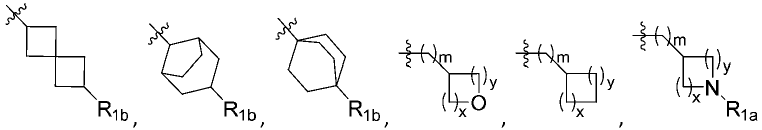 or pyridyl, and R₁ is optionally further substituted with 1 to 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
m is 0 or 1; and
x and y are each independently 1, 2 or 3.

In one or more embodiments of the present application,
R₀ is C₁₋₄ alkyl, wherein the C₁₋₄ alkyl is optionally further substituted with one or more substituents selected from halogen and deuterium; and
R₁ is

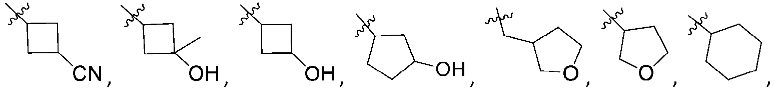

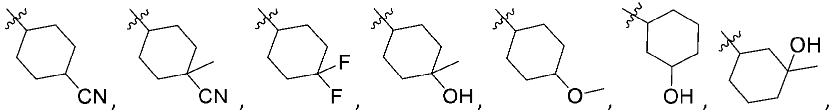

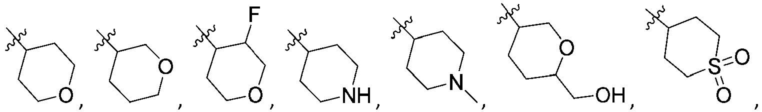

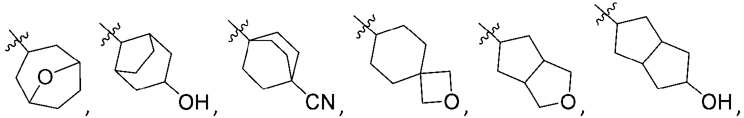

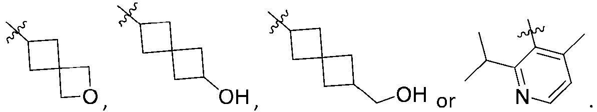

In one or more embodiments of the present application, the active ingredient is selected from:

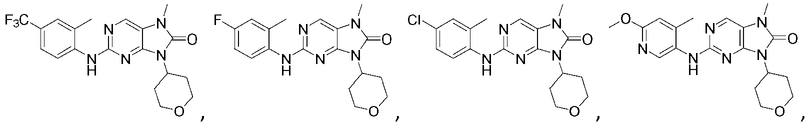

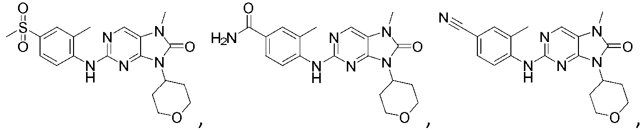

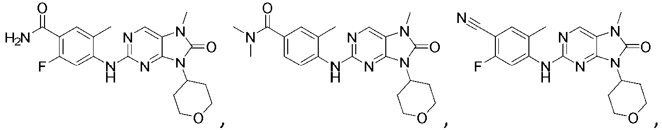

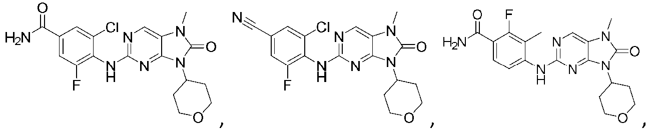

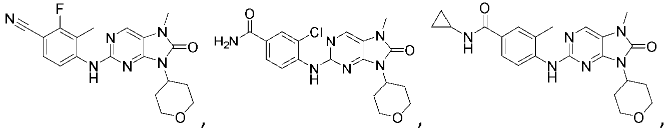

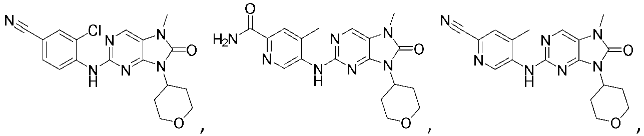

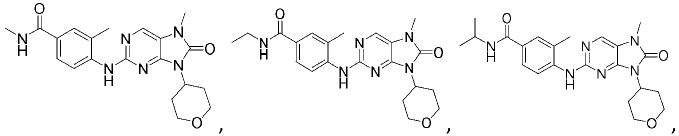

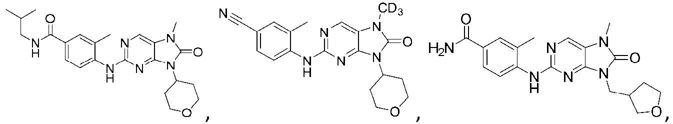

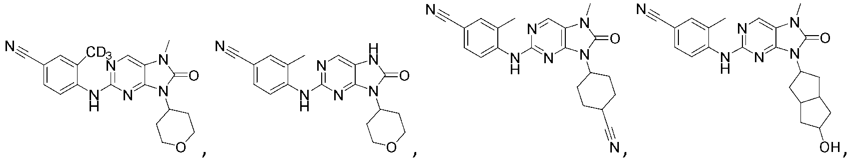

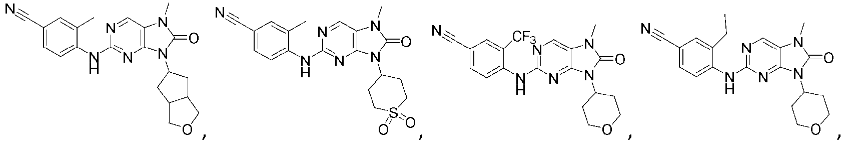

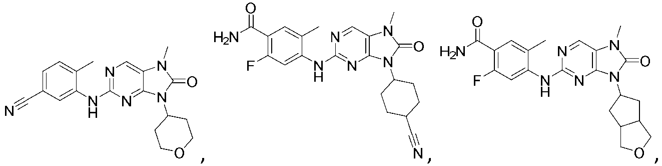

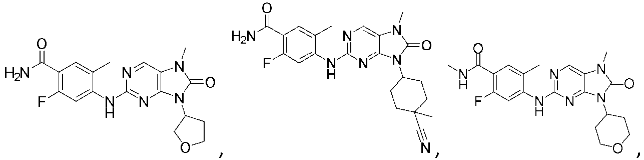

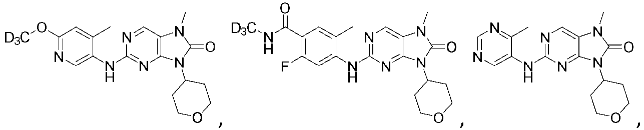

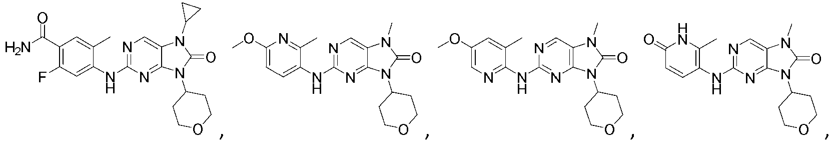

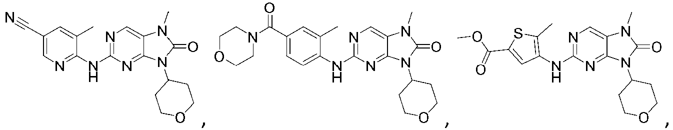

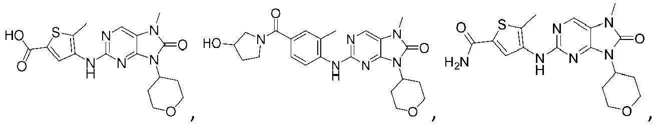

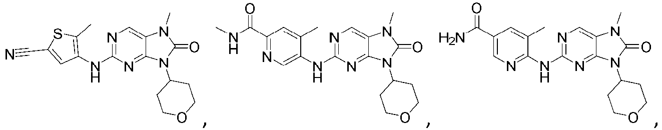

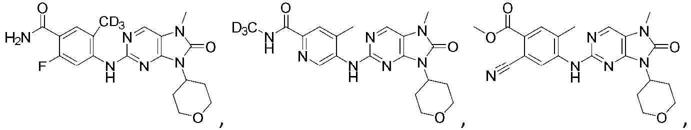

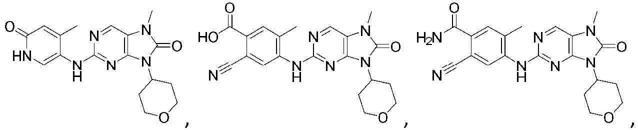

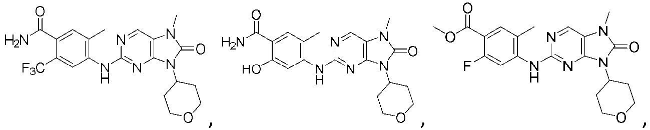

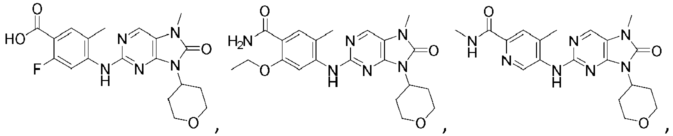

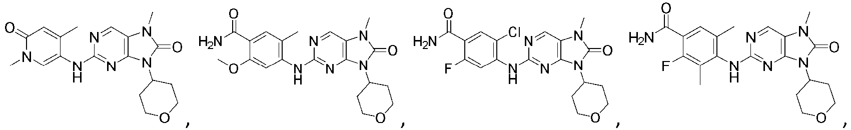

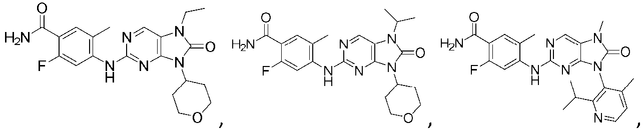

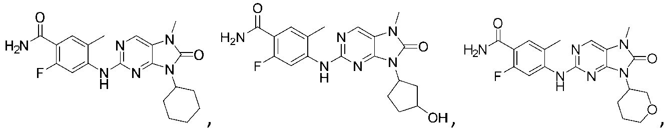

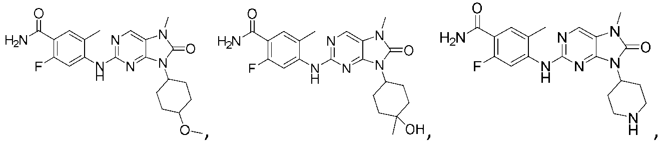

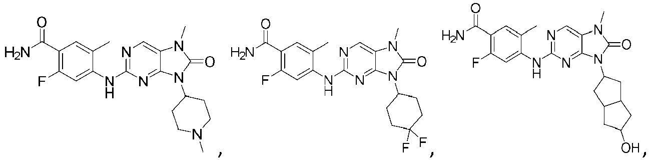

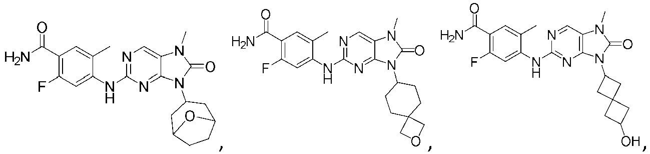

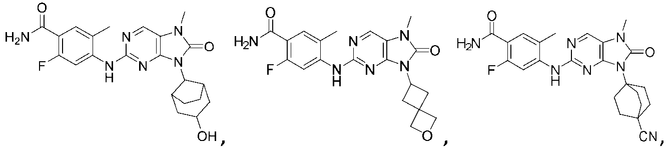

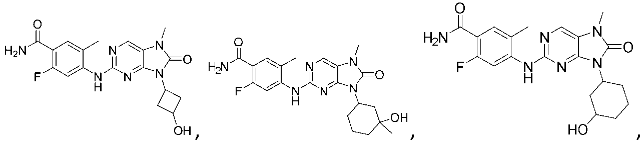

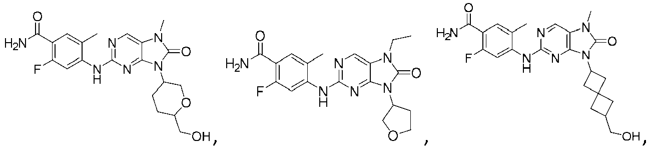

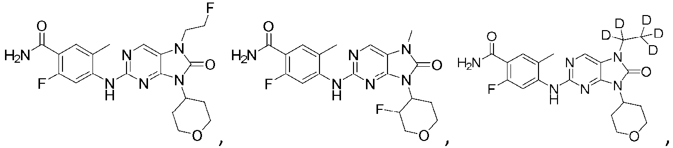

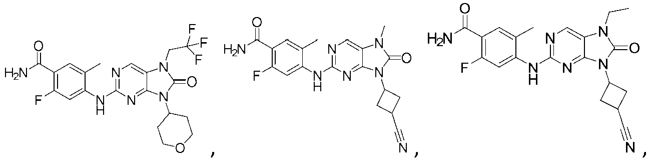

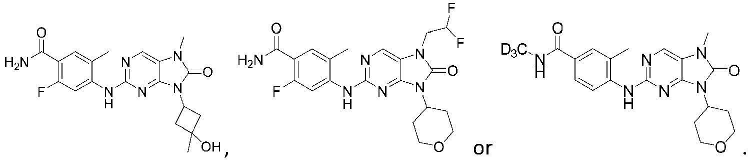

In other embodiments of the present application, among the aforementioned tumors, the leukemia is selected from acute lymphocitic leukemia or acute myeloblastic leukemia, and the acute myeloblastic leukemia comprises myeloblastic leukemia, promyelocytic leukemia, myelomonocytic leukemia, monocytic leukemia and/or erythroleukemia; the chronic leukemia comprises chronic myelogenous/granulocytic leukemia and/or chronic lymphocitic leukemia; the lymphoma comprises Hodgkin lymphoma and/or non-Hodgkin lymphoma; the gastric cancer comprises gastric adenocarcinoma; the head and neck cancer comprises tongue adenoid cystic carcinoma and/or adenoid cystic carcinoma; the lung cancer comprises pulmonary large-cell neuroendocrine carcinoma, lung adenocarcinoma, pulmonary sarcomatoid carcinoma, and/or lung squamous cell carcinoma; the cervical cancer comprises cervical squamous cell carcinoma; the breast cancer comprises invasive ductal carcinoma and ductal carcinoma; and the ovarian cancer comprises ovarian clear cell carcinoma or ovarian serous carcinoma.

The present application further provides a method for treating a tumor of esophageal cancer, lung cancer, head and neck cancer, gastric cancer, pleural mesothelioma, thymic carcinoma, kidney cancer, bladder cancer, hepatocellular carcinoma, colorectal cancer, lymphoma, ovarian cancer, breast cancer, fibrosarcoma, myosarcoma, liposarcoma, chondrosarcoma, osteoblastic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, pancreatic cancer, prostate cancer, squamous cell carcinoma, basal cell carcinoma, adenocarcinoma, sweat gland carcinoma, sebaceous carcinoma, papillary carcinoma, papillary adenocarcinoma, cystadenocarcinoma, medullary carcinoma, bronchogenic carcinoma, hepatocellular carcinoma, cholangiocarcinoma, choriocarcinoma, seminoma, embryonal carcinoma, Wilms' tumor, cervical cancer, testicular tumor, small cell lung cancer, epithelial cancer, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma, leukemia, chronic leukemia, polycythemia vera, multiple myeloma, Waldenstrom's macroglobulinemia or heavy chain disease, or any combination thereof, comprising oral administration in combination with radiotherapy: orally administering a therapeutically effective amount of a DNA-PK inhibitor, which is continuously administered daily or periodically until disease progression or toxicity is unacceptable and is orally administered before or after radiotherapy, wherein the DNA-PK inhibitor has a structure of the active ingredient as described previously.

In one or more embodiments of the present application, on the day of radiotherapy, a DNA-PK inhibitor is orally administered before or after radiotherapy.

In one or more embodiments of the present application, on the day of each radiotherapy, the DNA-PK inhibitor is orally administered once a day on an empty stomach 1-2 hours before radiotherapy, and the administration is accompanied by radiotherapy, i.e., the administration occurs on the day of radiotherapy and not on days without radiotherapy.

In one or more embodiments of the present application, the periodic administration comprises a cycle of 7 days consisting of 5 consecutive days of administration, followed by 2 days of discontinuation before entering the next cycle of administration.

In one or more embodiments of the present application, the daily dose of the active ingredient is 25-600 mg, preferably 50-400 mg, more preferably 100-300 mg.

In one or more embodiments of the present application, the daily dose of the active ingredient is 25 mg, or 50 mg, or 75 mg.

### BRIEF DESCRIPTION OF THE DRAWINGS

FIG. 1 shows a compound A.
FIG. 2 shows a graph of the trend of the volume of tumor in a BALB/c mouse model bearing a CT26 transplanted tumor.
FIG. 3 shows a graph of the trend of body weight change in a BALB/c mouse model bearing a CT26 transplanted tumor.

### DETAILED DESCRIPTION OF EMBODIMENTS

The implementation process and beneficial effects of the present invention are described in detail below by way of specific examples, which are intended to help readers better understand the essence and characteristics of the present invention but not to limit the scope of implementation of the present invention.

The present invention will be described in further detail below with reference to the accompanying drawings.

The compound A in the examples is compound 62 of WO 2021209055 and is prepared according to the preparation method therefor.

### Inhibition experiment using BALB/c mouse model bearing CT26 transplanted tumor

### 1. Experimental steps

Female BALB/c mice were selected to constitute a CT26 subcutaneously transplanted tumor model by subcutaneous injection of tumor cells. When the mean tumor volume reached 150-200 mm³, mice were divided into 3 experimental groups: Vehicle, IR, and (Compound A (20 mpk) + IR), respectively, with 8 animals per group and 24 animals in total for grouping experiments. Each irradiated group was given an irradiation dose of 2 Gy for 5 consecutive days, the administration group was given the corresponding drug once a day for 39 consecutive days, and the Vehicle group was given a vehicle.

### 2. Assay method

2.1 Tumor volume: During a drug treatment cycle, the long diameter (a) and short diameter (b) of tumor tissue were measured using an electronic vernier caliper to calculate the volume of the tumor (tumor volume = 0.5 × a × b²).

2.2 Body weight of mice: Mice were weighed at least twice a week during a drug treatment cycle.

### 3. Experimental results

### 3.1 Tumor volume results

**Table 1. Tumor volume statistics (Mean ± SEM, mm³)**

| **Treatment** | **D0** | **D4** | **D7** | **D11** | **D14** | **D18** |
|---|---|---|---|---|---|---|
| Vehicle | 167 ± 25 | 307 ± 59 | 475 ± 80 | 804 ± 137 | 1230 ± 255 | 2073 ± 428 |
| IR | 168 ± 24 | 298 ± 56 | 342 ± 62 | 315 ± 80** | 234 ± 61*** | 267 ± 83*** |
| Compound A (20 mpk) + IR | 168 ± 22 | 260 ± 41 | 284 ± 47* | 253 ± 56*** | 155 ± 28*** | 101 ± 33***# |

| **Treatment** | **D21** | **D25** | **D28** | **D32** | **D35** | **D39** |
|---|---|---|---|---|---|---|
| Vehicle | / | / | / | / | / | / |
| IR | 361 ± 115 | 520 ± 148 | 724 ± 196 | 1068 ± 309 | 1520 ± 418 | 2251 ± 634 |
| Compound A (20 mpk) + IR | 93 ± 34# | 169 ± 71# | 288 ± 105# | 462 ± 148# | 605 ± 189# | 828 ± 247# |

| | | | | | | |
|---|---|---|---|---|---|---|
| Notes: the day of grouping is D0; * indicates P ≤ 0.05 compared to animals in the Vehicle group; ** indicates P ≤ 0.01 compared to animals in the Vehicle group; *** indicates *P* ≤ 0.001 compared to animals in the Vehicle group; # indicates P ≤ 0.05 compared to animals in the IR group; mpk is mg/kg; and On D18, since the tumor in some mice in the Vehicle group was too large, the mice in this group were euthanized considering animal ethics issues. | | | | | | |

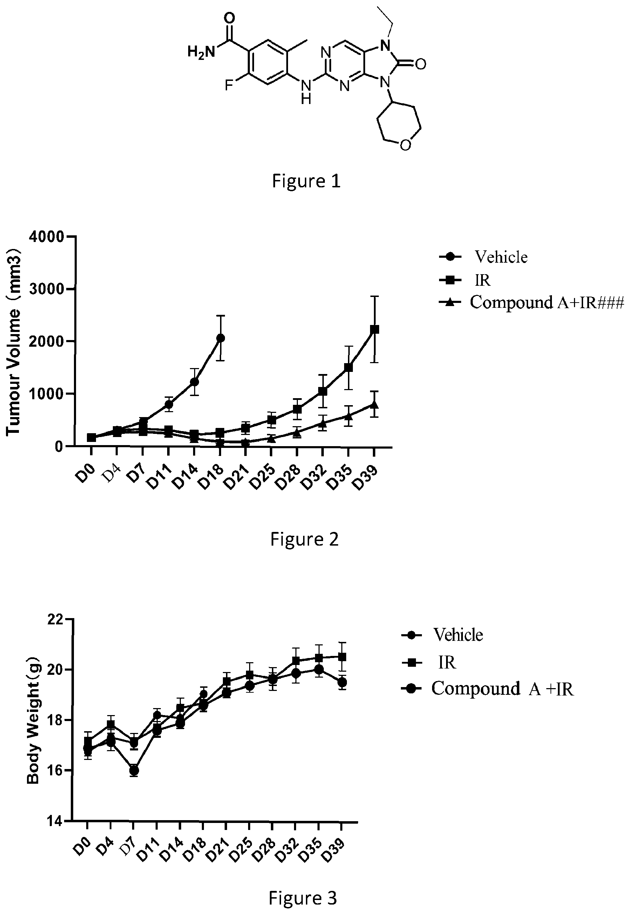

As shown in Table 1 and FIG. 2, the tumor volume of the group treated with compound A in combination with IR was significantly reduced compared to the Vehicle and IR groups, and after four days of treatment, the difference in volume between the group treated with compound A in combination with IR and the IR group gradually increased, indicating that the compound A can significantly enhance the anti-tumor efficacy of radiotherapy.

### 3.2 Body weight results

**Table 2. Body weight statistics**

| **Treatment** | **D0** | **D4** | **D7** | **D11** | **D14** | **D18** |
|---|---|---|---|---|---|---|
| Vehicle | 16.7 ± 0.3 | 17.3 ± 0.3 | 17.1 ± 0.3 | 18.2 ± 0.3 | 18.1 ± 0.3 | 19.1 ± 0.3 |
| IR | 17.2 ± 0.4 | 17.8 ± 0.4 | 17.2 ± 0.3 | 17.7 ± 0.4 | 18.5 ± 0.4 | 18.7 ± 0.3 |
| Compound A (20 mpk) + IR | 16.8 ± 0.3 | 17.1 ± 0.3 | 16.0 ± 0.2 | 17.6 ± 0.2 | 17.9 ± 0.2 | 18.6 ± 0.2 |

| **Treatment** | **D21** | **D25** | **D28** | **D32** | **D35** | **D39** |
|---|---|---|---|---|---|---|
| Vehicle | / | / | / | / | / | / |
| IR | 19.6 ± 0.4 | 19.8 ± 0.5 | 19.7 ± 0.4 | 20.4 ± 0.5 | 20.5 ± 0.5 | 20.6 ± 0.6 |
| Compound A (20 mpk) + IR | 19.1 ± 0.2 | 19.4 ± 0.3 | 19.7 ± 0.2 | 19.9 ± 0.4 | 20.1 ± 0.3 | 19.6 ± 0.3 |

| | | | | | | |
|---|---|---|---|---|---|---|
| Notes: mpk is mg/kg; and On D18, since the tumor in some mice in the Vehicle group was too large, the mice in this group were euthanized considering animal ethics issues. | | | | | | |

As shown in Table 2 and FIG. 3, the body weight of mice in the Vehicle group was stable during the experiment; the body weight of the IR group was slightly decreased during irradiation and began to rise after irradiation; and at the end of the experiment on D39, there is no significant difference in mean body weight between the IR group and the group treated with compound A in combination with IR, indicating that the treatment of compound A in combination with radiotherapy had no effect on body weight gain of animals and the animals had good tolerance.

### Clinical evaluation

Experiment purpose: evaluating the safety, tolerability and preliminary efficacy of drug of compound A in combination with radiotherapy in patients with malignant solid tumors.

Clinical subjects: at an age of 18 years old (minimum age) to unlimited (maximum age) gender: male or female; and who is histologically or cytologically diagnosed with a solid tumor that has indications for radiotherapy after evaluation by an oncologist. Radiotherapy techniques such as IMRT, IGRT, VMAT, TOMO, SBRT or SRS can be used.

### Experiment scheme:

Usage and amount: 25 mg, 50 mg, 75 mg, 100 mg, 150 mg, 200 mg, 250 mg, 300 mg, 400 mg, 500 mg, and 600 mg. On the day of each radiotherapy, the study drug (a drug of compound A) is orally administered once a day on an empty stomach 1-2 hours before radiotherapy, and the administration is accompanied by radiotherapy (the administration occurs on the day of radiotherapy and not on days without radiotherapy). Course of medication: until the end of radiotherapy or the subject is not tolerant to the study drug and/or radiotherapy, etc.

### Endpoint criteria:

**Table 3 Endpoint criteria**

| | Criteria | Evaluation time |
|---|---|---|
| Primary endpoint criteria and evaluation time | 1. DLT, AE, etc. | 2 weeks of screening period, DLT observation or treatment period (every 7 days during radiotherapy, at the end of radiotherapy, 2 weeks after the end of radiotherapy, and 4 weeks after the end of radiotherapy), safety follow-up (every 6 weeks after the end of radiotherapy until 48 weeks after the end of radiotherapy or until subjects withdraw from the study due to disease progression or other reasons, whichever occurs first). |
| | 2. BOR in radiotherapy field; ORR, DCR, DOR, and progression-free survival; and overall survival. | |
| Secondary endpoint criteria and evaluation time | 1. PK parameters: including but not limited to Cmax; AUC; etc. | 2 weeks of screening period, DLT observation or treatment period (every 7 days during radiotherapy, at the end of radiotherapy, 2 weeks after the end of radiotherapy, and 4 weeks after the end of radiotherapy), safety follow-up (every 6 weeks after the end of radiotherapy until 48 weeks after the end of radiotherapy or until subjects withdraw from the study due to disease progression or other reasons, whichever occurs first). |
| | 2. Study endpoint: BOR in radiotherapy field; ORR, DCR, DOR, and progression-free survival; and overall survival. | |

### Evaluation results of clinical efficacy

**Table 4 Evaluation of efficacy in subject**

| Age | Gender | Height | Body weight |
|---|---|---|---|
| 58 years old | Male | 169 cm | 73.3 kg |
| Diagnosed tumor type | Pulmonary large-cell neuroendocrine carcinoma (LCNEC) | | |
| Primary tumor site | Left lung | | |
| Time since tumor diagnosis | 7 months | | |
| Prior treatment | Left lung and mediastinal lymph node focal radiotherapy: 60Gy/15f | | |
| | Sequential chemotherapy: Etoposide + Carboplatin, 6 cycles | | |
| TNM Staging | TxN3M1, IVb | | |
| Treatment regimens | Every 7 days is a treatment cycle, during which the treatment of radiotherapy and oral administration of drugs is conducted daily for 5 consecutive days, with a dose per fraction of 3Gy and capsules of compound A (25 mg) administered orally once a day on an empty stomach 1-2 hours before radiotherapy on the day of each radiotherapy; in addition, the administration is accompanied by radiotherapy, which does not occur on days without radiotherapy. After 5 consecutive days of treatment, there is a two-day break before entering the next cycle of treatment. Except in cases where there is a need to interrupt radiotherapy for special reasons. | | |
| Best of response (BOR) | In the radiotherapy field: SD or Non-CR/Non-PD | | |
| Dose limiting toxicity (DLT) | No | | |
| Serious adverse event (SAE) | No | | |

The clinical study results show that the growth of tumor in the radiotherapy field is greatly inhibited for the patient with pulmonary large-cell neuroendocrine carcinoma during clinical trial cycles, with good treatment safety; additionally, for other indications, including prostate cancer, pleural mesothelioma, etc., the growth of tumors in patients in the radiotherapy field is all significantly inhibited, and the safety and tolerability of the treatment process are both excellent.

Specific embodiments are described in detail in the description of the present invention. A person skilled in the art should recognize that the embodiments described above are exemplary and cannot be construed as limiting the present invention. Additionally, a person skilled in the art can make several improvements and modifications to the present invention without departing from the principle of the present invention, and the technical solutions obtained based on these improvements and modifications also fall within the scope of protection of the claims of the present invention.

## Claims

1. Use of a compound represented by formula (I), or a stereoisomer, solvate, prodrug, metabolite, pharmaceutically acceptable salt or co-crystal thereof as an active ingredient in the preparation of a drug for use in the treatment of a tumor in combination with radiotherapy, wherein the tumor is selected from esophageal cancer, lung cancer, head and neck cancer, gastric cancer, pleural mesothelioma, thymic carcinoma, kidney cancer, bladder cancer, hepatocellular carcinoma, colorectal cancer, lymphoma, ovarian cancer, breast cancer, fibrosarcoma, myosarcoma, liposarcoma, chondrosarcoma, osteoblastic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, pancreatic cancer, prostate cancer, squamous cell carcinoma, basal cell carcinoma, adenocarcinoma, sweat gland carcinoma, sebaceous carcinoma, papillary carcinoma, papillary adenocarcinoma, cystadenocarcinoma, medullary carcinoma, bronchogenic carcinoma, hepatocellular carcinoma, cholangiocarcinoma, choriocarcinoma, seminoma, embryonal carcinoma, Wilms' tumor, cervical cancer, testicular tumor, small cell lung cancer, epithelial cancer, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma, leukemia, chronic leukemia, polycythemia vera, multiple myeloma, Waldenstrom's macroglobulinemia or heavy chain disease, or any combination thereof: 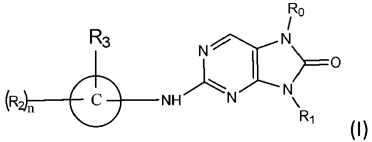 wherein 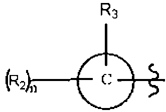 is 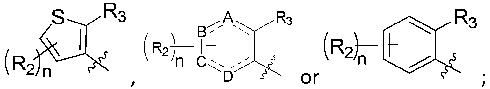
-̅ -̅ -̅ is a single bond or double bond;
in 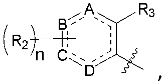 A, B, C, D are each independently C or N, and at least one of A, B, C and D is N;
R₀ is H, C₁₋₆ alkyl or cyclopropyl, wherein the C₁₋₆ alkyl is optionally further substituted with one or more substituents selected from halogen and deuterium;
R₁ is 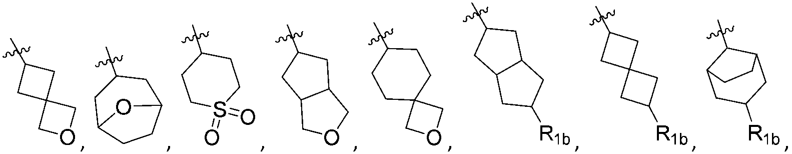 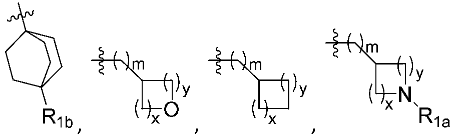 or pyridyl, and R₁ is optionally further substituted with 1 or 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
R₂ is H, cyano, =O, carboxyl, -C(=O)NR₂ₐR_{2b}, C₁₋₆ alkoxy, C₁₋₆ alkyl, halogen, -S(=O)₂R₂ₐ or - C(=O)OC₁₋₆ alkyl, wherein the C₁₋₆ alkyl, -C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atoms to which they are attached form a 5- to 6-membered heterocyclyl, which contains 1, 2 or 3 heteroatoms selected from N, O and S and is optionally further substituted with one or more substituents selected from C₁₋₆ alkyl, OH and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1 or 2; and
x and y are each independently 1, 2 or 3;
with the provisos that
when 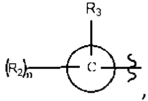 R₀, R₂, and R₃ simultaneously satisfy the following conditions, R₁ is not 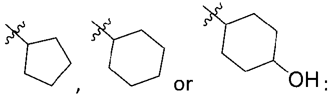 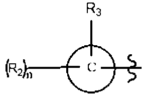 is 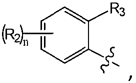 n is 1, R₀ is H or methyl, R₂ is methoxy or -S(=O)₂Me, and R₃ is methyl.

2. The use according to claim 1, wherein the active ingredient is the compound of formula (I), or a stereoisomer, solvate, prodrug, metabolite, pharmaceutically acceptable salt or co-crystal thereof, wherein
R₁ is 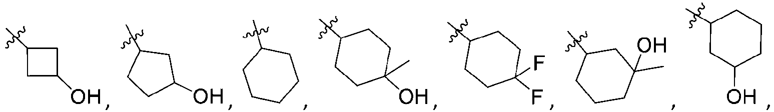 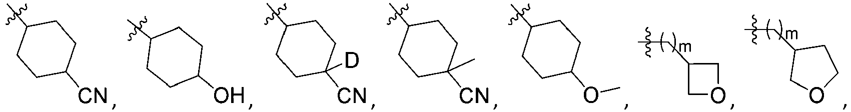 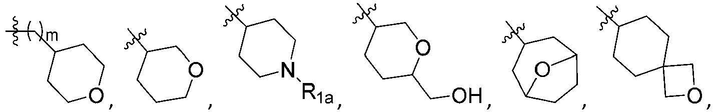 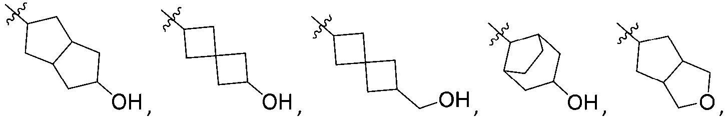 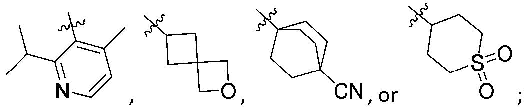
R₁ₐ is H or C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl, wherein the -C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D or halogen;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from OH and halogen; R₃ is halogen or C₁₋₆ alkyl, wherein the C₁₋₆ alkyl is optionally further substituted with 1 to 3 substituents selected from D and halogen;
m is 0 or 1; and
n is 0, 1 or 2;
with the provisos that
when 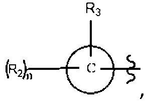 R₀, R₂, and R₃ simultaneously satisfy the following conditions, R₁ is not 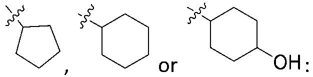 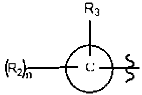 is 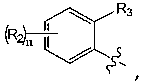 n is 1, R₀ is H or methyl, R₂ is methoxy or -S(=O)₂Me, and R₃ is methyl.

3. The use according to claim 1, wherein the active ingredient is the compound of formula (I), or a stereoisomer, solvate, prodrug, metabolite, pharmaceutically acceptable salt or co-crystal thereof, wherein 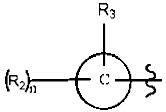 is 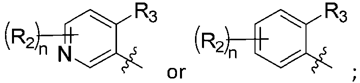
R₀ is H, C₁₋₄ alkyl or cyclopropyl, wherein the C₁₋₄ alkyl is optionally further substituted with one or more substituents selected from halogen and D;
R₁ is 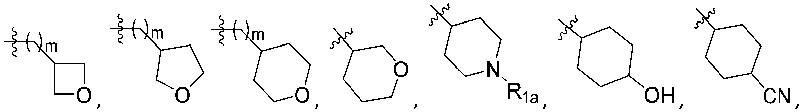 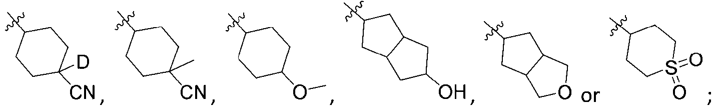
R₁ₐ is H, C₁₋₆ alkyl or -C(=O)C₁₋₆ alkyl;
R₂ is H, cyano, -C(=O)NR₂ₐR_{2b}, C₁₋₆ alkoxy, halogen, -S(=O)₂R₂ₐ or -C(=O)OC₁₋₆ alkyl, wherein the -C(=O)OC₁₋₆ alkyl or C₁₋₆ alkoxy is optionally substituted with one or more substituents selected from halogen and deuterium;
R₂ₐ and R_{2b} are each independently H, C₁₋₆ alkyl, or 3- to 5-membered cycloalkyl, wherein the C₁₋₆ alkyl is optionally further substituted with one or more substituents selected from OH, D and halogen;
alternatively, R₂ₐ and R_{2b} together with the atoms to which they are attached form a 5- to 6-membered heterocyclyl, which contains 1 to 3 heteroatoms selected from N, O and S and is also optionally further substituted with one or more substituents selected from OH and halogen;
R₃ is halogen or C₁₋₆ alkyl, wherein the C₁₋₆ alkyl is optionally further substituted with 1 to 3 substituents selected from D and halogen;
m is 0 or 1; and
n is 0, 1 or 2;
with the provisos that
when 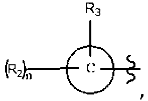 R₀, R₂, and R₃ simultaneously satisfy the following conditions, R₁ is not 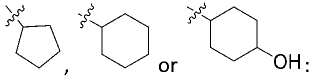
when 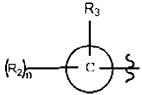 is 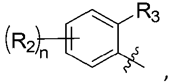 n is 1, R₀ is H or methyl, R₂ is methoxy or - S(=O)₂Me, and R₃ is methyl.

4. The use according to claim 1, wherein the active ingredient is selected from a compound of formula (II), or a stereoisomer, solvate, prodrug, metabolite, pharmaceutically acceptable salt or co-crystal thereof: 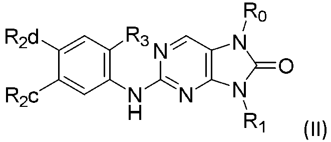 wherein
R₀ is H, C₁₋₆ alkyl or cyclopropyl, wherein the C₁₋₆ alkyl is optionally further substituted with one or more substituents selected from halogen and deuterium;
R₁ is 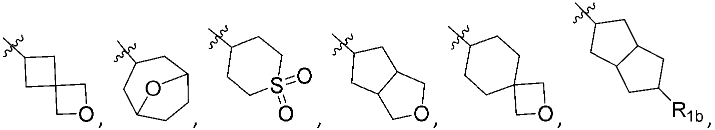 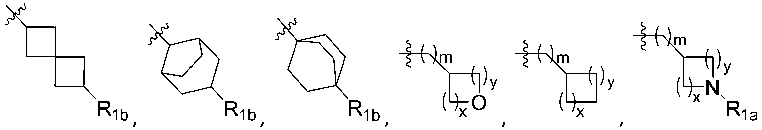 or pyridyl, and R₁ is optionally further substituted with 1 to 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
R_{2c} is H, cyano, halogen or C₁₋₆ alkoxy;
R_{2d} is H, cyano, carboxyl, -C(=O)NR₂ₐR_{2b}, C₁₋₆ alkyl, halogen, -S(=O)₂R₂ₐ or -C(=O)OC₁₋₆ alkyl, wherein the C₁₋₆ alkyl and -C(=O)OC₁₋₆ alkyl is optionally substituted with one or more substituents selected from halogen and deuterium;
R₂ₐ and R_{2b} are H, C₁₋₆ alkyl, or 3- to 5-membered cycloalkyl, wherein the C₁₋₆ alkyl is optionally further substituted with one or more substituents selected from OH, D, halogen, C₁₋₆ alkyl and C₁₋₆ alkoxy;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from C₁₋₆ alkyl, OH and halogen;
R₃ is halogen or C₁₋₆ alkyl, wherein the C₁₋₆ alkyl is optionally further substituted with 1 to 3 substituents selected from D and halogen;
m is 0 or 1;
n is 0, 1 or 2; and
x and y are each independently 1, 2 or 3;
with the provisos that
when 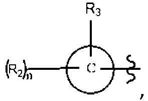 R₀, R₂, and R₃ simultaneously satisfy the following conditions, R₁ is not 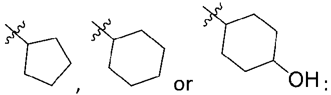 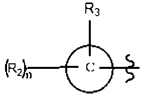 is selected from 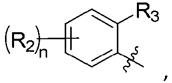 n is 1, R₀ is H or methyl, R₂ is methoxy or - S(=O)₂Me, and R₃ is methyl.

5. The use according to claim 4, wherein the active ingredient is selected from a compound of formula (III), or a stereoisomer, solvate, prodrug, metabolite, pharmaceutically acceptable salt or co-crystal thereof: 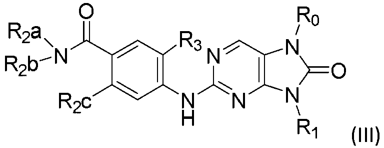 wherein
R₀ is H, C₁₋₆ alkyl or cyclopropyl, wherein the C₁₋₆ alkyl is optionally further substituted with one or more substituents selected from halogen and deuterium;
R₁ is 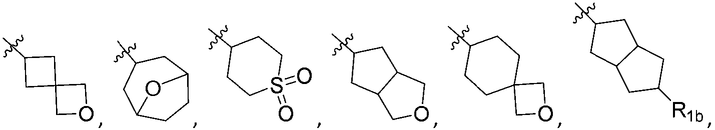 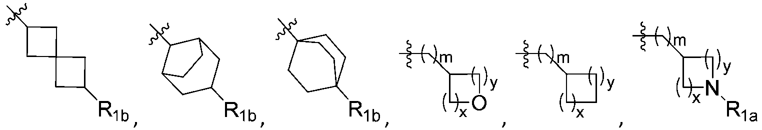 or
pyridyl, and R₁ is optionally further substituted with 1 to 2 substituents selected from D, halogen, cyano, hydroxyl, C₁₋₆ alkyl and C₁₋₆ alkoxy;
R_{1b} is H, OH, cyano, or hydroxyl substituted C₁₋₆ alkyl;
R₂ₐ and R_{2b} are each independently H, C₁₋₆ alkyl, or 3- to 5-membered cycloalkyl, wherein the C₁₋₆ alkyl is optionally further substituted with one or more substituents selected from D or halogen;
alternatively, R₂ₐ and R_{2b} together with the atoms to which they are attached form a 5- to 6-membered heterocyclyl, which contains 1 to 3 heteroatoms selected from N, O and S and is optionally further substituted with one or more substituents selected from C₁₋₆ alkyl, OH and halogen;
R_{2c} is H, cyano, halogen or C₁₋₆ alkoxy, wherein the C₁₋₆ alkoxy is optionally substituted with one or more deuterium;
R₃ is halogen or C₁₋₆ alkyl, wherein the C₁₋₆ alkyl is optionally further substituted with 1 to 3 substituents selected from D or halogen;
m is 0 or 1;
n is 0, 1 or 2; and
x and y are each independently 1, 2 or 3.

6. The use according to claim 1, wherein the active ingredient is selected from a compound of formula (IV), or a stereoisomer, solvate, prodrug, metabolite, pharmaceutically acceptable salt or co-crystal thereof: 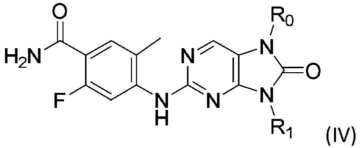 wherein
R₀ is H, C₁₋₆ alkyl or cyclopropyl, wherein the C₁₋₆ alkyl is optionally further substituted with one or more substituents selected from halogen and deuterium; and
R₁ is -(CH)ₘ-4- to 7-membered carbocyclyl, -(CH)ₘ-4- to 7-membered heterocyclyl, -(CH)ₘ-8-to 12-membered bridged ring, -(CH)ₘ-7- to 12-membered spiro ring, wherein the -(CH)ₘ-4-to 7-membered carbocyclyl, -(CH)ₘ-4- to 7-membered heterocyclyl, -(CH)ₘ-8- to 12-membered bridged ring, or -(CH)ₘ-7- to 12-membered spiro ring is optionally further substituted with one or more substituents selected from hydroxy, cyano, halogen, =O, C₁₋₆ alkyl, C₁₋₆ alkoxy, and hydroxy substituted C₁₋₆ alkyl.

7. The use according to claim 6, wherein the active ingredient is selected from the compound of formula (IV), or a stereoisomer, solvate, prodrug, metabolite, pharmaceutically acceptable salt or co-crystal thereof, wherein
R₀ is H, C₁₋₆ alkyl or cyclopropyl, wherein the C₁₋₆ alkyl is optionally further substituted with one or more substituents selected from halogen and deuterium;
R₁ is 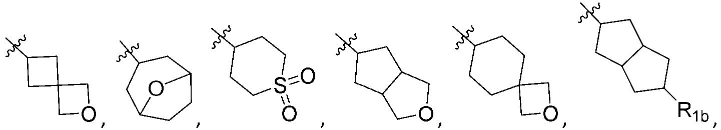 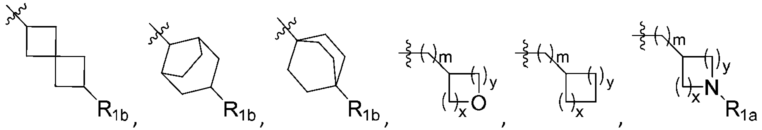 or pyridyl, and R₁ is optionally further substituted with 1 to 2 substituents selected from D, halogen, cyano, hydroxyl, C₁₋₆ alkyl and C₁₋₆ alkoxy;
R₁ₐ is H or C₁₋₆ alkyl;
R_{1b} is H, OH, cyano, or hydroxyl substituted C₁₋₆ alkyl;
m is 0 or 1; and
x and y are each independently 1, 2 or 3.

8. The use according to claim 7, wherein the active ingredient is the compound of formula (IV), or a stereoisomer, solvate, prodrug, metabolite, pharmaceutically acceptable salt or co-crystal thereof, wherein
R₀ is C₁₋₄ alkyl, wherein the C₁₋₄ alkyl is optionally further substituted with one or more substituents selected from halogen and deuterium; and
R₁ is

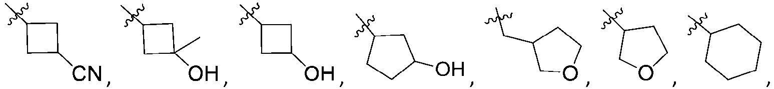

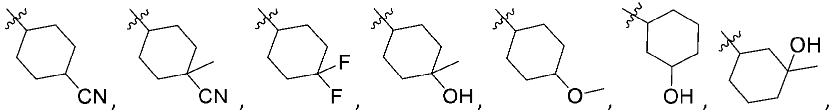

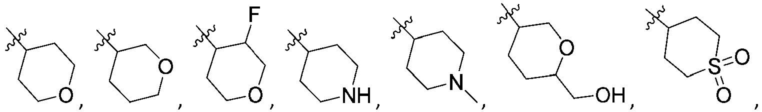

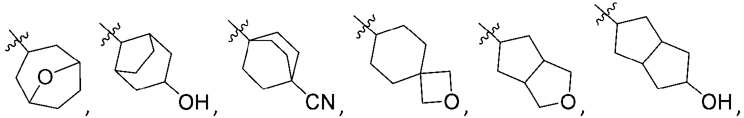

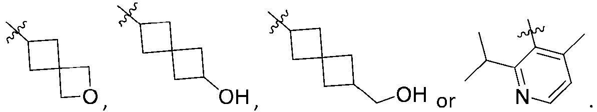

9. The use according to any one of claims 1-8, wherein the active ingredient is selected from: 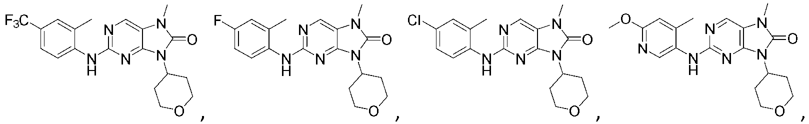 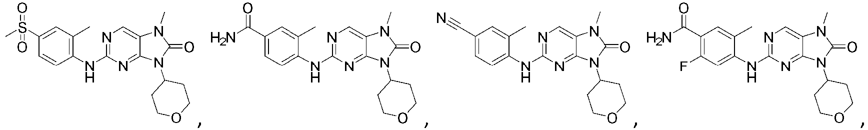 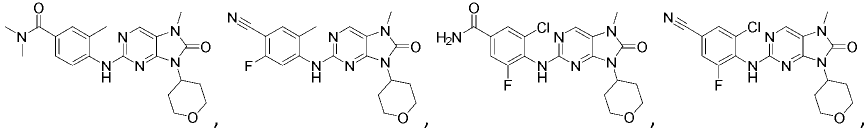 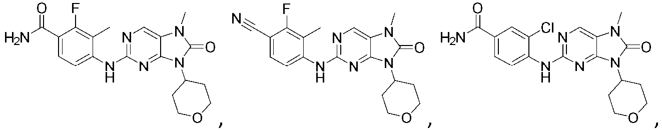 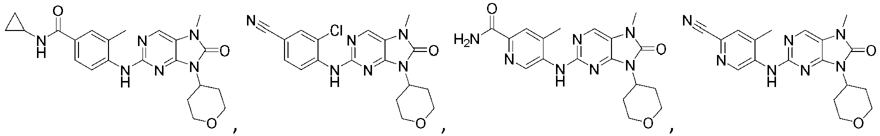 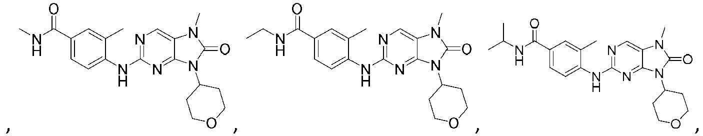 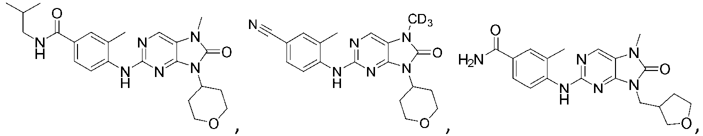 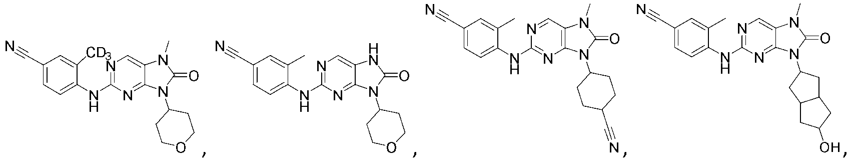 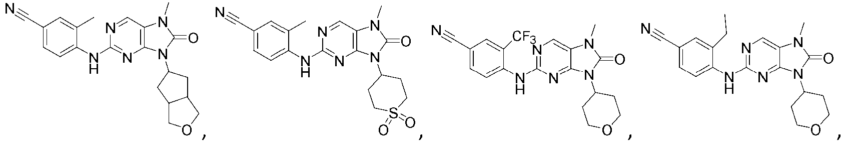 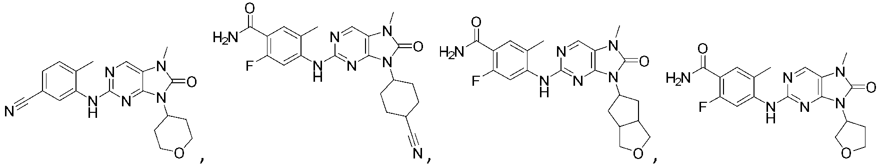 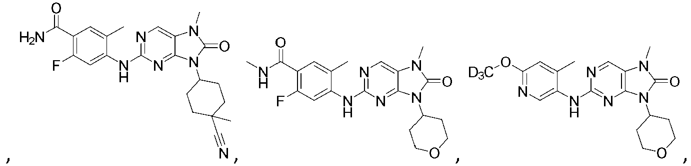 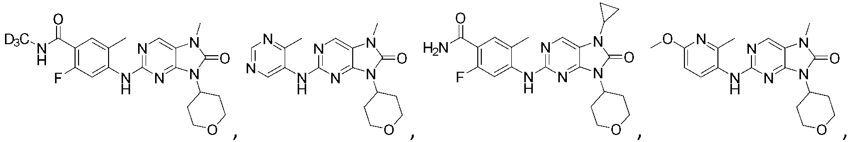 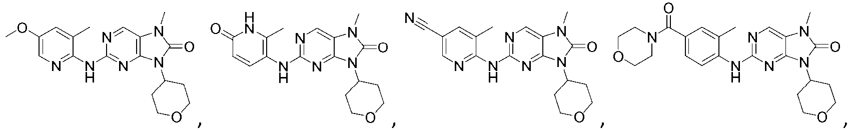 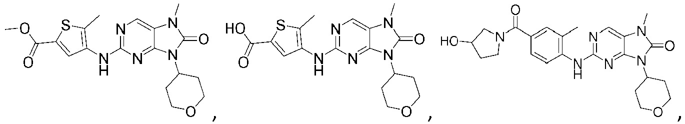 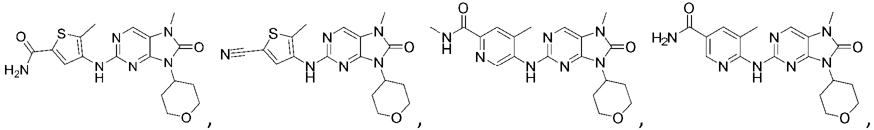 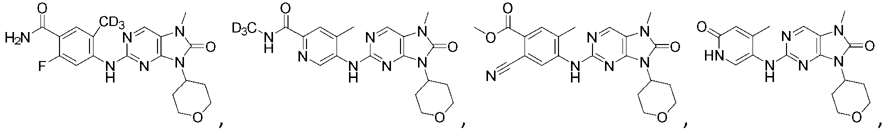 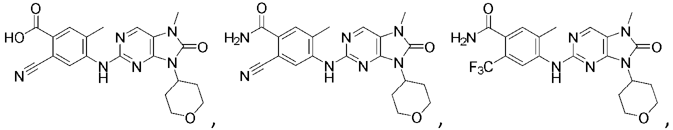 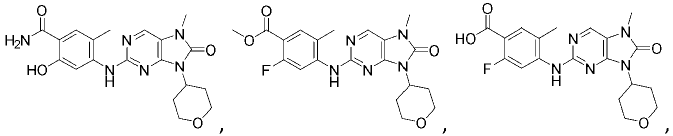 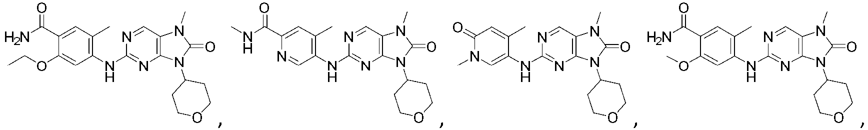 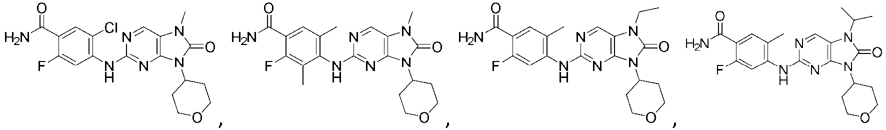 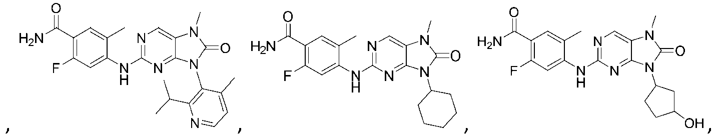 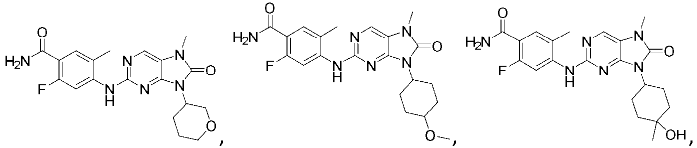 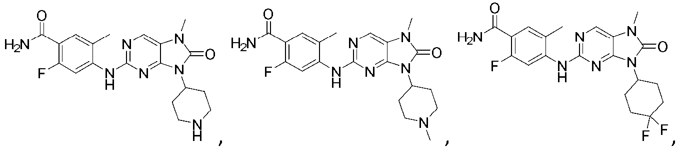 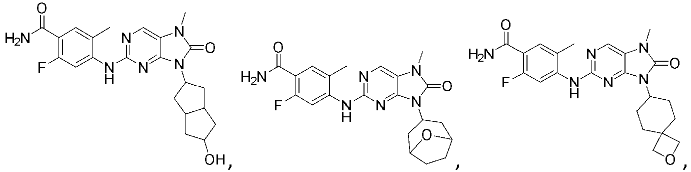 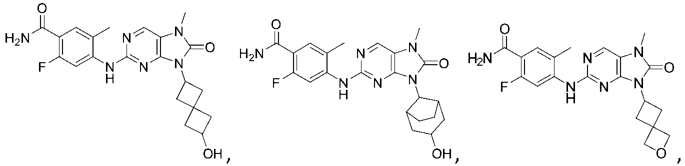 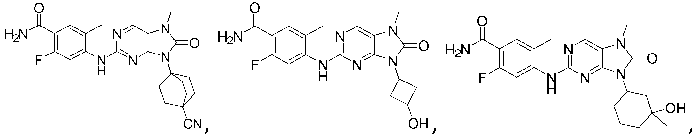 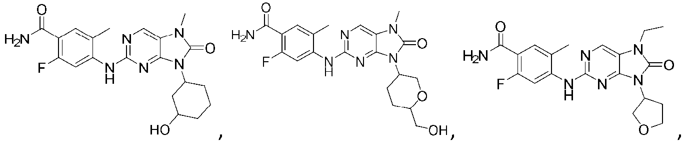 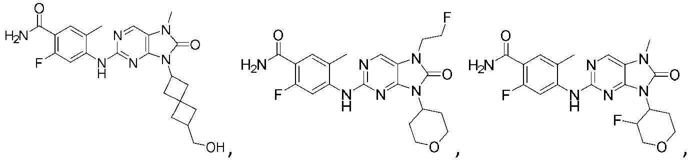 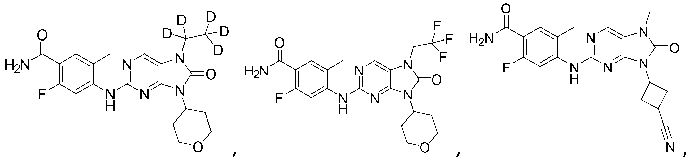 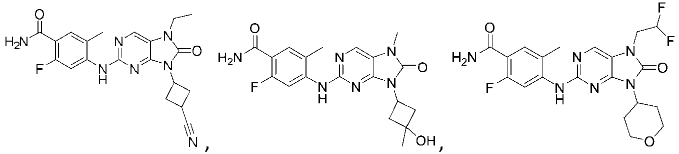 or

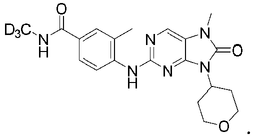

10. The use according to any one of claims 1-9, wherein the leukemia is selected from acute lymphocitic leukemia or acute myeloblastic leukemia, and the acute myeloblastic leukemia comprises myeloblastic leukemia, promyelocytic leukemia, myelomonocytic leukemia, monocytic leukemia and/or erythroleukemia; the chronic leukemia comprises chronic myelogenous/granulocytic leukemia and/or chronic lymphocitic leukemia; the lymphoma comprises Hodgkin lymphoma and/or non-Hodgkin lymphoma; the gastric cancer comprises gastric adenocarcinoma; the head and neck cancer comprises tongue adenoid cystic carcinoma and/or adenoid cystic carcinoma; the lung cancer comprises pulmonary large-cell neuroendocrine carcinoma, lung adenocarcinoma, pulmonary sarcomatoid carcinoma, and/or lung squamous cell carcinoma; the cervical cancer comprises cervical squamous cell carcinoma; the breast cancer comprises invasive ductal carcinoma and ductal carcinoma; and the ovarian cancer comprises ovarian clear cell carcinoma or ovarian serous carcinoma.

11. A method for treating a tumor of esophageal cancer, lung cancer, head and neck cancer, gastric cancer, pleural mesothelioma, thymic carcinoma, kidney cancer, bladder cancer, hepatocellular carcinoma, colorectal cancer, lymphoma, ovarian cancer, breast cancer, fibrosarcoma, myosarcoma, liposarcoma, chondrosarcoma, osteoblastic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, pancreatic cancer, prostate cancer, squamous cell carcinoma, basal cell carcinoma, adenocarcinoma, sweat gland carcinoma, sebaceous carcinoma, papillary carcinoma, papillary adenocarcinoma, cystadenocarcinoma, medullary carcinoma, bronchogenic carcinoma, hepatocellular carcinoma, cholangiocarcinoma, choriocarcinoma, seminoma, embryonal carcinoma, Wilms' tumor, cervical cancer, testicular tumor, small cell lung cancer, epithelial cancer, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma, leukemia, chronic leukemia, polycythemia vera, multiple myeloma, Waldenstrom's macroglobulinemia or heavy chain disease or any combination thereof, comprising: oral administration in combination with radiotherapy: orally administering a therapeutically effective amount of a DNA-PK inhibitor, which is continuously administered daily or periodically until disease progression or toxicity is unacceptable and is orally administered before or after radiotherapy, wherein the DNA-PK inhibitor has a structure of the active ingredient as defined in any one of claims 1-9.

12. The method according to claim 11, wherein on the day of radiotherapy, the DNA-PK inhibitor is orally administered before or after radiotherapy.

13. The method according to claim 11 or 12, wherein on the day of each radiotherapy, the DNA-PK inhibitor is orally administered once a day on an empty stomach 1-2 hours before radiotherapy, and the administration is accompanied by radiotherapy, i.e., the administration occurs on the day of radiotherapy and not on days without radiotherapy.

14. The method according to claim 13, wherein the periodic administration comprises a cycle of 7 days consisting of 5 consecutive days of administration, followed by 2 days of discontinuation before entering the next cycle of administration.

15. The use according to any one of claims 1 to 10 and the method according to any one of claims 11 to 14, wherein the daily dose of the active ingredient is 25-600 mg, preferably 50-400 mg, more preferably 100-300 mg.

16. The use according to any one of claims 1 to 10 and the method according to any one of claims 11 to 14, wherein the daily dose of the active ingredient is 25 mg, or 50 mg, or 75 mg.
